# EUROPÄISCHE PATENTANMELDUNG

(11) **EP 4 368 720 A1**
(43) Veröffentlichungstag der Anmeldung: **15.05.2024**
(21) Anmeldenummer: 22206345.5
(22) Anmeldetag: 09.11.2022
(51) Int. Cl.: C12P 7/20, C12P 7/24, C12P 7/40, C12P 7/56, C12N 9/04, C12N 9/88

(54) **VERFAHREN ZUR HERSTELLUNG EINER WÄSSERIGEN LÖSUNG, WELCHE GLYCERIN ENTHÄLT**

(71) Anmelder: Annikki GmbH, 8074 Raaba-Grambach (AT)
(72) Erfinder: Staunig, Nicole, 8074 Raaba-Grambach (AT); Dupont, Maria, 8074 Raaba-Grambach (AT)
(74) Vertreter: Schwarz & Partner Patentanwälte GmbH

(57) **Zusammenfassung**

Verfahren zur Herstellung einer wässerigen Lösung, welche Glycerin und ein Salz der Brenztraubensäure oder der Milchsäure enthält, indem ein Salz der Gluconsäure und/oder der Galactonsäure, in einer wässerigen, Alkohol-hältigen Lösung in vitro mit einem Enzymsystem, welches eine Dehydratase, eine Aldolase, eine Alkoholdehydrogenase samt Kofaktor, eine Glyceraldehyd-Reduktase samt Kofaktor und gegebenenfalls eine Lactat-Dehydrogenase samt Kofaktor umfasst, behandelt wird, wodurch eine Glycerin- und ein Salz der Brenztraubensäure oder der Milchsäure hältige wässerige Lösung erhalten wird, aus welcher das Enzymsystem abgetrennt und welche gegebenenfalls aufkonzentriert wird. (Figur)

## Beschreibung

Die Erfindung betrifft ein Verfahren zur Herstellung einer wässerigen Lösung, welche Glycerin und ein Salz der Brenztraubensäure (Pyruvat) oder der Milchsäure (Lactat) enthält.

Wässerige Lösungen, welche Glycerin und Pyruvat bzw. Lactat enthalten, werden für die Herstellung diverser Kosmetikprodukte verwendet. Darüber hinaus ist Pyruvat ein wichtiger Baustein für die Synthese von Chemikalien wie Alkoholen oder Aminosäuren.

Durch Reduktion von Pyruvat gelangt man zu Milchsäure bzw. Lactat, welches über einen breiten Anwendungsbereich verfügt. Milchsäure wird z.B. als Säuerungsmittel in der Lebensmittelindustrie, als Entkalkungsmittel, pH-Regulator oder Reinigungsmittel in der chemischen Industrie, als Zusatz in Anti-Akne-Cremes oder Feuchtigkeitscremes in der kosmetischen Industrie sowie als Precursor für Acrylsäure oder Ethyllactat verwendet (Wee et al., 2006). Darüber hinaus kann Milchsäure auch zum Hautpeeling (*Chemisches Peeling*) eingesetzt werden (Smith, 1996). Die bifunktionelle Milchsäure lässt sich des Weiteren auch polymerisieren, wobei Polylactid (PLA), ein bioabbaubarer und biokompatibler Kunststoff, entsteht, der Anwendungen in der Verpackungsindustrie, Textilindustrie, im Elektroniksowie im Medizinbereich hat (Balla et al., 2021).

In den letzten Jahren hat sich das Interesse auf Prozesse verlagert, welche hochselektive Umsätze mittels Enzymkaskadenreaktionen nutzen, um Zielchemikalien in einem Schritt (*Eintopf* oder *One-Pot)* zu erhalten. Die EP2700714A1, die US8859247B2 und die EP2204453B1 beschreiben ein Portfolio an Kaskadenreaktionen, in welcher Glucose in einer Kaskade aus fünf enzymatischen Umsetzungen zu zwei Molekülen Pyruvat umgewandelt wird. Von Glucose ausgehend folgt die Kaskade dem nichtphosphorylierenden Entner-Doudoroff-Pfad zu D-Glyceraldehyd und Pyruvat (Zuckeroxidation, Dehydratisierung und Aldolspaltung). D-Glyceraldehyd wird danach weiter zu D-Glycerat oxidiert und anschließend zu Pyruvat dehydratisiert. Dieses dient dann als Plattform für weitere Transformationen zu einer Reihe an Aminosäuren und Alkoholen. Die Enzyme der Kaskade umfassen dabei eine Glucose-Dehydrogenase, eine promiskuitive Dihydroxysäure-Dehydratase (katalysiert Dehydratisierungen von Gluconat und D-Glycerat), eine 2-Keto-3-deoxygluconat-Aldolase und eine Aldehyd-Dehydrogenase. Bei Verwendung einer ebenfalls promiskuitiven Dehydrogenase, welche sowohl Glucose als auch D-Glyceraldehyd als Substrat akzeptiert, kann das System auf drei Enzyme minimiert werden.

Der in der EP2700714A1 beschriebene Prozess zur Bildung von Pyruvat aus Glucose wurde von Sutiono et al. aufgegriffen und um Glycerin als Startmaterial erweitert. Die Umwandlung von Glucose zu zwei Molekülen Pyruvat erfolgt in fünf Schritten (siehe vorhergehender Absatz). Glycerin muss zuerst zu D-Glyceraldehyd oxidiert werden, welches dann in zwei Schritten zu Pyruvat umgewandelt wird. Das Hauptaugenmerk dieser Studie lag in der Charakterisierung einer promiskuitiven Dehydratase aus *Paralcaligenes ureilyticus,* welche sowohl die Dehydratisierung von Gluconat als auch von D-Glycerat ermöglicht. Mit einer Enzymkaskade, welche die besagte Dehydratase beinhaltet, konnten 25 mM Glucose (4,5 g/l) in Gegenwart von 5 mM NAD⁺ zu ca. 45 mM Ethanol (= 90% Umsatz) innerhalb von 12 h umgewandelt werden (Sutiono et al., 2020).

Durch Reduktion gelangt man von D-Glyceraldehyd zu Glycerin. Aus Glycerin lassen sich wertvolle Folgeprodukte, wie z.B. 1,3-Propandiol, welches in der Synthese von Polymeren, bei der Herstellung von Kosmetika oder in Schmierstoffen Anwendung findet, oder Acrolein (2-Propenal), einen Ausgangsstoff für Acrylsäure oder deren Ester, herstellen. Glycerin selbst wird z.B. in Kosmetika, Lebensmittel und Pharmazeutika eingesetzt.

Glycerin und Milchsäure dienen ferner als Ausgangsstoffe für die Synthese von Glycerin-Lactat-Ester (wie Glycerintrilactat), die als Nahrungsergänzungsmittel während körperlicher Betätigung und anschließender Regeneration eingesetzt werden können, da Milchsäure als Energiequelle für Herz und Skelettmuskel dient (US 6743821 B2).

Wie oben bereits angeführt, finden sowohl Glycerin als auch Milchsäure (und zum Teil auch Brenztraubensäure) Anwendung in Kosmetikprodukten, wie z.B. Hautpeelings (Smith, 1996; Prestes et al., 2013; Ghersetich et al., 2004; O'Connor et al., 2018) oder Salben (*Apotheke des Universitätsklinikums Heidelberg,* 2015). Zur Herstellung solcher Produkte werden die einzelnen Reinstoffe miteinander vermischt.

Glycerin kann durch Hydrolyse von Triglyceriden mit Alkalilaugen (Verseifung) oder Wasser hergestellt werden (Tan et al., 2013; Rodrigues et al., 2017). Des Weiteren sind aber auch fermentative Verfahren zur Herstellung von Glycerin (z.B. ausgehend von Glucose oder Saccharose) bekannt (z.B. Wang et al., 2001; Aslankoohi et al., 2015; US 8129170 B1).

Großtechnisch fällt Glycerin hauptsächlich als Nebenprodukt der Biodiesel-Herstellung an, bei der Fette und Öle mit Methanol umgesetzt werden und dabei Fettsäuremethylester und Glycerin bilden (Tan et al., 2013; Rodrigues et al., 2017).

Die Verwendung von Methanol hat einen prinzipiellen Nachteil: es wird heutzutage zu einem großen Teil aus Erdgas (ca. 65%) und Erdöl (ca. 35%) produziert, also aus nicht erneuerbaren Kohlenstoffquellen (nur ca. 0,2% stammt aus erneuerbaren Ressourcen (*IRENA* & *METHANOL INSTITUTE,* 2021)). Die heutige großtechnische Herstellung von Glycerin ist also auf den Verbrauch nicht erneuerbarer Kohlenstoffquellen angewiesen.

Hier setzt nun die vorliegende Erfindung an und setzt sich zur Aufgabe, ein Verfahren zur Herstellung einer wässerigen Lösung enthaltend Glycerin und ein Salz der Brenztraubensäure oder der Milchsäure, wie es zum Beispiel in der Kosmetikindustrie gebraucht wird, zur Verfügung zu stellen, welches ausschließlich auf erneuerbaren Kohlenstoffquellen beruht und darüber hinaus auch als "vegan" bezeichnet werden kann, was insbesondere bei der Anwendung der wässerigen Lösung in der Kosmetikindustrie von Bedeutung ist.

### Detaillierte Beschreibung der Erfindung

Diese Aufgabe wird erfindungsgemäß gelöst, indem ein Salz der Gluconsäure und/oder der Galactonsäure, in einer wässerigen, Alkohol-hältigen Lösung in vitro mit einem Enzymsystem, welches eine Dehydratase, eine Aldolase, eine Alkoholdehydrogenase samt Kofaktor, eine Glyceraldehyd-Reduktase samt Kofaktor und gegebenenfalls eine Lactat-Dehydrogenase samt Kofaktor umfasst, behandelt wird, wodurch eine Glycerin- und ein Salz der Brenztraubensäure oder der Milchsäure hältige wässerige Lösung erhalten wird, aus welcher das Enzymsystem abgetrennt und welche gegebenenfalls aufkonzentriert wird.

In dieser Patentanmeldung werden die Begriffe D-Glucose austauschbar mit Glucose, D-Gluconat austauschbar mit Gluconat, Gluconat austauschbar mit Gluconsäure, D-Galactonat austauschbar mit Galactonat, Galactonat austauschbar mit Galactonsäure, D-Glycerinaldehyd austauschbar mit D-Glyceraldehyd bzw. Glyceraldehyd sowie Milchsäure austauschbar mit Lactat verwendet.

Gluconsäure (bzw. deren Anion Gluconat) ist eine von der Glucose abstammende Zuckersäure, die eine wichtige Industriechemikalie darstellt. Glucose wiederum kommt in gebundener Form (Cellulose) in Biomasse vor und ist das häufigste Monosaccharid in der Natur. Generell enthalten Strohe, Hölzer oder Abfallprodukte aus der Lebensmittelindustrie, wie Getreide-Spreu (z.B. Hafer-Spelzen) Cellulosen und Hemicellulosen, aus welchen Monomer-Zucker in wechselnder Zusammensetzung freigesetzt werden können, vornehmlich die Hexosen Glucose, Mannose und Galactose, die Pentosen Xylose und Arabinose, sowie von den Zuckern abgeleitete Säuren (z.B. Glucuronsäure). In Mais-Hüllen beispielsweise findet man, bezogen auf den Gesamtzucker, 64% Glucose, 4% Galactose, 2% Mannose, 10% Arabinose und 16% Xylose (Hromádková & Ebringerová, 1995), in Weizenstroh hingegen sind es 56% Glucose, 1% Galactose, 2% Mannose, 5% Arabinose und 30% Xylose (Collins et al., 2014). Methoden zur effizienten Trennung der C₆- und C₅-Zucker erlauben es, diese Stoffstromtypen getrennt voneinander weiter zu behandeln (z.B. WO 2011/014894).

Gluconat ist durch Oxidation an der C₁-Position der Glucose chemisch leicht zugänglich. Die fermentative Herstellung von Gluconat mittels *Aspergillus niger* oder *Gluconobacter oxydans* stellt die wichtigste Technik dar. Gluconat kann aber auch enzymatisch unter Verwendung der Glucose-Oxidase aus Glucose gewonnen werden, wobei Luftsauerstoff als Oxidationsmittel dient. Das entstehende Wasserstoffperoxid ist schädlich für das eingesetzte Enzym und muss daher z.B. durch den Einsatz von Katalase entfernt werden (Kornecki et al., 2020).

In Organismen ist Gluconat ein wichtiges Intermediat im sogenannten Entner-Doudoroff-Weg (ED-Weg), bei dem ausgehend von Glucose zwei Pyruvat-Moleküle und ein Adenosintriphosphat-Molekül (ATP) gebildet werden. Die genaue Ausprägungsform des ED-Wegs hängt vom Mikroorganismus ab, er tritt beispielsweise in Bakterien wie *Escherichia coli* auf. Für Archaeen sind weitere Abwandlungen wie der nicht-phosphorylierende, semi-phosphorylierende oder verzweigte ED-Weg bekannt. Der Kernschritt in allen Wegen ist die Aldolspaltung einer C₆-Zuckersäure (2-Keto-3-deoxygluconat bzw. 2-Keto-3-deoxy-6-phosphogluconat je nach Mikroorganismus) in zwei C₃-Produkte (Pyruvat und Glycerinaldehyd bzw. Glycerinaldehyd-3-phosphat) (Entner & Doudoroff, 1952; Conway, 1992; Peekhaus & Conway, 1998; Reher et al., 2010; Sutter et al., 2016).

Im ED-Weg wird das Intermediat D-Glyceraldehyd ausschließlich zur D-Glycerinsäure oxidiert, welches dann über mehrere Schritte weiter zum Pyruvat umgewandelt wird. Pyruvat ist ein zentrales Intermediat im zellulären Stoffwechsel und als solches ein wichtiger Ausgangsstoff für die Herstellung von Alkoholen, Aminosäuren oder 2,3-Butandiol (Sutiono et al., 2020), was den ED-Weg für biosynthetische Anwendungen interessant macht. Pyruvat kann aber auch zur Behandlung von Sonnen-geschädigter Gesichtshaut in Form von Peelings eingesetzt werden (Ghersetich et al., 2004).

Durch die Promiskuität der Enzyme des Entner-Doudoroff-Weges kann nicht nur Glucose, sondern auch Galactose (das C₄-Epimer) von denselben Enzymen zu Pyruvat und D-Glyceraldehyd umgewandelt werden. Dies ist sehr gut für das hyperthermophile Archaeon *Sulfolobus solfataricus* belegt (Lamble et al., 2003; Theodossis et al., 2004).

Seitdem wurde eine Reihe weiterer Prozesse beschrieben, um Chemikalien enzymatisch zu produzieren. So werden Alkoholdehydrogenasen (ADH) für die Produktion hochwertiger chiraler Alkohole genutzt, wobei der Kofaktor NAD z. B. durch Zugabe von Glucose und Glucose-Dehydrogenase regeneriert wird (Goldberg et al., 2007). Generell werden Glucose- oder Formiat-Dehydrogenasen und Alkoholdehydrogenasen für das Recycling des Kofaktors verwendet (Schrittwieser et al., 2018).

Die so erhaltenen Produktlösungen (Glycerin + Salz der Brenztraubensäure bzw. Milchsäure) dienen als Basis für die Herstellung von diversen Kosmetikprodukten und basieren vollständig auf erneuerbaren Rohstoffen (C₆-Zuckersäuren gewonnen aus in Biomasse vorkommenden Monosacchariden) und sind darüber hinaus vegan. Somit umgeht das Verfahren die Verwendung von Methanol aus fossilen Rohstoffen zur Herstellung von Glycerin.

Es hat sich somit überaschenderweise gezeigt, dass Aufgabe der vorliegenden Erfindung gelöst werden kann, wenn die Umsetzung nicht fermentativ durchgeführt wird, sondern die Enzyme als solche in der wässerigen Lösung enthalten sind, also in vitro gearbeitet wird.

Der Begriff "Enzymsystem" bezeichnet die Gesamtheit der eingesetzten Enzyme, also die Dehydratase, die Aldolase, die Glyceraldehyd-Reduktase samt Kofaktor, die Lactat-Dehydrogenase samt Kofaktor sowie eine Alkoholdehydrogenase, zum Beispiel eine NAD-spezifische Alkoholdehydrogenase und/oder eine NADP-spezifische Alkoholdehydrogenase. Diese Enzyme samt den Kofaktoren können zum Beispiel als solche (Enzymisolate) der wässerigen Lösung zugegeben werden. Es ist jedoch auch möglich, Zellen, welche die angegebenen Enzyme exprimieren, in der wässerigen Lösung zu suspendieren. Je nach Reaktionsbedingungen bleiben die Zellen intakt oder platzen auf und geben das Enzym in die wässrige Umgebung ab. Bei Vorliegen von intakten Zellen diffundieren die Substrate in die Zelle, wo sie vom Enzym umgesetzt werden, und die Produkte im Anschluss wieder heraus. In diesem Fall wird unter dem Begriff "Enzymsystem" eine Zellsuspension verstanden. Ferner ist unter dem Begriff "Enzymsystem" ein Homogenat zu verstehen, welches aus der Zellsuspension erhalten werden kann, indem die Zellen z.B. Ultraschall ausgesetzt werden, um sie zum Platzen zu bringen. Schließlich sind unter dem Begriff "Enzymsystem" auch Lysate zu verstehen, die aus den Homogenaten erhalten werden, indem die festen Zellbestandteile abgetrennt werden.

Ein weiterer Vorteil des erfindungsgemäßen Verfahrens besteht darin, dass die Umsetzung auch bei nichtphysiologischen Bedingungen, wie etwa hohen Substratkonzentrationen, vorgenommen werden kann, also bei Konzentrationen, unter denen ein fermentatives Verfahren nicht arbeiten kann.

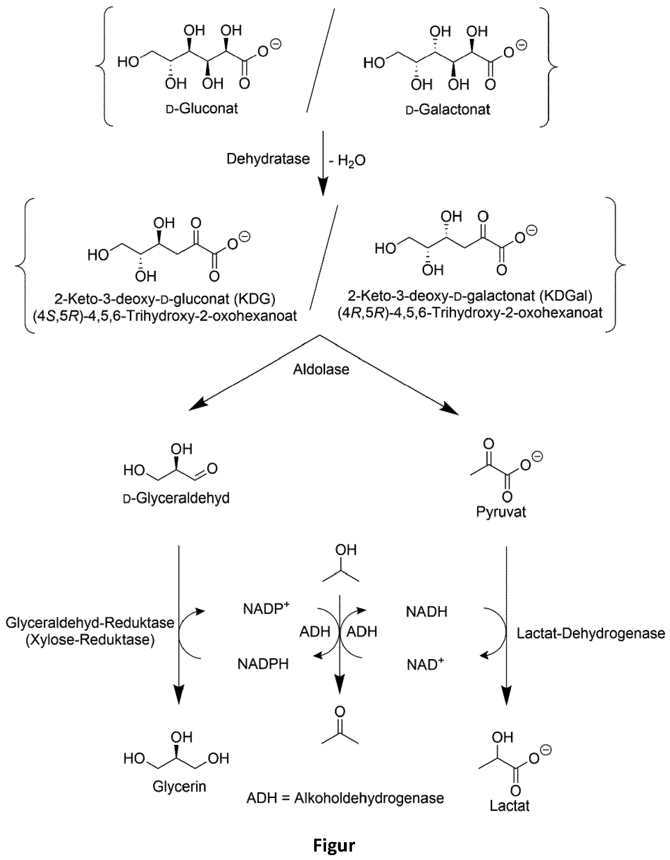

Das Reaktionsschema ist in der beiliegenden Figur dargestellt.

Gemäß einer bevorzugten Ausführungsform des erfindungsgemäßen Verfahrens, wird ein Alkalisalz, insbesondere das Natriumsalz oder das Kaliumsalz der Gluconsäure und der Galactonsäure eingesetzt.

In weiteren bevorzugten Ausführungsformen des erfindungsgemäßen Verfahrens beträgt die Konzentration des Alkalisalzes der Gluconsäure und/oder der Galactonsäure in der wässerigen Lösung zwischen 50 und 350 g/l, 150 und 300 g/l und noch mehr bevorzugt zwischen 150 und 250 g/l.

Zusätzlich ist bevorzugt, dass das erfindungsgemäße Verfahren bei einer Temperatur zwischen 20 und 50 °C, zwischen 25 und 40 °C und noch mehr bevorzugt zwischen 30 und 40 °C durchgeführt wird.

Der besonders bevorzugte pH-Bereich der Reaktion liegt zwischen 7,5 und 8,5.

Bei einer weiteren, bevorzugten Variante des erfindungsgemäßen Verfahrens liegen die das Enzymsystem bildenden Enzyme in einer Suspension, im Homogenat und/oder im Lysat der entsprechenden, sie bildenden Zellen vor. Es hat sich gezeigt, dass bei Verwendung von Suspensionen, Lysaten und/oder Homogenaten die Kofaktoren der Dehydrogenasen und Reduktasen nicht extra zugesetzt werden müssen, weil sie in der Suspension, im Lysat und/oder im Homogenat bereits enthalten sind.

Suspension bedeutet in diesem Kontext eine Suspension von *resting cells.* Diese werden nach der Kultivierung geerntet (vom Nährmedium abgetrennt) und in einem geeigneten Puffersystem suspendiert. Im Gegensatz zu fermentativen Verfahren, bei denen auch mit ganzen Zellen gearbeitet wird, können die *resting cells* aufgrund der Entfernung von Kohlenstoff-Quellen und Nährstoffen nicht mehr wachsen, sondern dienen nur der Umsetzung von Substraten (Lin & Tao, 2017). Homogenat steht in diesem Kontext für eine physikalisch und/oder chemisch behandelte Suspension (z.B. mittels Druckes, Lysozym oder Ultraschall behandelt), wobei die Zellbestandteile aus den Zellen freigesetzt werden. Ein Lysat wird erhalten, wenn die unlöslichen Zellbestandteile des Homogenats beispielsweise durch Filtration oder Zentrifugation entfernt werden (siehe *Produktion Enzyme* für Details).

Das erfindungsgemäße Verfahren umfasst die Umwandlungen von Gluconat bzw. Galactonat mit Reaktionen des Entner-Doudoroff-Weges (Dehydratisierung und Aldolspaltung) sowie einer bzw. zwei abschließenden Reduktionen, bei denen das Kofaktor-Recycling mit einer bzw. zwei Alkoholdehydrogenasen und einem Alkohol, bevorzugt Isopropanol (2-Propanol), als Kosubstrat erfolgt (siehe Figur).

Um die Umsetzung beider C₆-Zuckersäuren zu ermöglichen, wird natürliche Enzympromiskuität genutzt, die es erlaubt, mit einem Enzym bis zu zwei Zielreaktionen zu katalysieren, um die Enzymkaskade zu minimieren.

Gemäß einem weiteren bevorzugten Aspekt der Erfindung finden alle Reaktionsschritte des Verfahrens sowie die Regeneration der Kofaktoren in einem einzigen Reaktionsgefäß statt *(Eintopfreaktion),* d.h. es wird die kostenintensive Isolierung der Intermediate vermieden. In der bevorzugten Ausführungsform werden alle Enzyme zu Beginn der Reaktion eingesetzt.

In einer besonders bevorzugten Ausführung des Verfahrens werden für die Konversion des Startmaterials nur Enzyme aus den Enzymgruppen Dehydrogenasen, Reduktasen, Dehydratasen und Aldolasen verwendet, wobei eines oder mehrere dieser Enzyme aus jeder dieser Gruppen ausgewählt werden.

Die Umwandlung von Gluconat über (4S,5R)-4,5,6-Trihydroxy-2-oxohexanoat (2-Keto-3-deoxygluconat = KDG) bzw. Galactonat über (4R,5R)-4,5,6-Trihydroxy-2-oxohexanoat (2-Keto-3-deoxygalactonat = KDGal) zu Pyruvat und D-Glyceraldehyd umfasst dabei eine Dehydratase und eine Aldolase.

Die im Verfahren verwendete Dehydratase kann aus einer der Gruppen EC 4.2.1.5 (Arabonat-Dehydratase), 4.2.1.6 (Galactonat-Dehydratase), 4.2.1.7 (Altronat-Dehydratase), 4.2.1.8 (Mannonat-Dehydratase), 4.2.1.9 (Dihydroxysäure-Dehydratase), 4.2.1.25 (L-Arabonat-Dehydratase), 4.2.1.39 (Gluconat-Dehydratase), 4.2.1.40 (Glucarat-Dehydratase), 4.2.1.42 (Galactarat-Dehydratase), 4.2.1.67 (D-Fuconat-Dehydratase), 4.2.1.68 (L-Fuconat-Dehydratase), 4.2.1.82 (Xylonat-Dehydratase), 4.2.1.140 (Gluconat/Galactonat-Dehydratase), 4.2.1.146 (L-Galactonat-Dehydratase), 4.2.1.156 (L-Talarat-Dehydratase), 4.2.1.158 (Galactarat-Dehydratase, D-*threo*-bildend) und 4.2.1.176 (L-Lyxonat-Dehydratase) stammen, wobei die Gruppe 4.2.1.25 (L-Arabonat-Dehydratase) besonders bevorzugt ist.

Die im Verfahren verwendete Aldolase kann aus einer der Gruppen EC 4.1.2.18 (2-Dehydro-3-deoxy-L-pentonat-Aldolase), 4.1.2.20 (2-Dehydro-3-deoxyglucarat-Aldolase), 4.1.2.21 (2-Dehydro-3-deoxy-6-phosphogalactonat-Aldolase), 4.1.2.28 (2-Dehydro-3-deoxy-D-pentonat-Aldolase), 4.1.2.29 (5-Dehydro-2-deoxyphosphogluconat-Aldolase), 4.1.2.51 (2-Dehydro-3-deoxy-D-gluconat-Aldolase), 4.1.2.52 (4-Hydroxy-2-oxoheptandioat-Aldolase), 4.1.2.53 (2-Keto-3-deoxy-L-rhamnonat-Aldolase), 4.1.2.54 (L-*threo*-3-Deoxy-hexylosonat-Aldolase), 4.1.2.55 (2-Dehydro-3-deoxy-phosphogluconat/2-Dehydro-3-deoxy-6-phosphogalactonat-Aldolase) sowie 4.1.3.39 (4-Hydroxy-2-oxovalerat-Aldolase) stammen, wobei die Gruppe 4.1.2.55 (2-Dehydro-3-deoxy-phosphogluconat/2-Dehydro-3-deoxy-6-phosphogalactonat-Aldolase) besonders bevorzugt ist.

Die zur Reduktion von D-Glyceraldehyd verwendete Reduktase (Glyceraldehyd-Reduktase) stammt bevorzugt aus der Gruppe der D-Xylose-Reduktasen (EC 1.1.1.307).

Die zur Reduktion von Pyruvat zu Lactat verwendeten Dehydrogenase stammt aus der Gruppe EC 1.1.1.27 (L-Lactat-Dehydrogenase).

Die zur Regenerierung der Kofaktoren eingesetzten Alkoholdehydrogenasen stammen bevorzugt aus den Gruppen EC 1.1.1.1 (NAD-abhängige ADH) oder EC 1.1.1.2 (NADP-abhängige ADH).

Die hier vorgestellte enzymatische Strategie minimiert die Anzahl der Enzyme und erlaubt somit einen hocheffizienten und kostengünstigen/preislich kompetitiven Bioproduktionsprozess.

Mit den nachfolgenden Beispielen werden bevorzugte Ausführungsformen der Erfindung noch näher beschrieben.

### Materialien

Natrium-Gluconat, 2-Keto-3-deoxygluconsäure-Lithium-Salz, D-Glyceraldehyd, Natrium-Pyruvat, Natrium-L-Lactat, Zinkchlorid, IPTG (Isopropyl-β-D-thiogalactopyranosid) sowie HEPES (2-(4-(2-Hydroxyethyl)-1-piperazinyl)-ethansulfonsäure) wurden von Sigma-Aldrich, Glycerin, Magnesiumchlorid-Hexahydrat, Kaliumdihydrogenphosphat, di-Kaliumhydrogenphosphat, NAD⁺, NADH-Dinatriumsalz, NADP⁺-Dinatriumsalz sowie Natriumdodecylsulfat (SDS) wurden von Carl Roth, NADPH-Tetranatriumsalz, Lysozym sowie Methanol wurden von PanReac AppliChem (ITW Reagents) und Triethanolamin wurde von Chem-Lab NV bezogen. Kalium-D-Galactonat wurde gemäß Literaturvorschrift (Moore & Link, 1940) aus Galactose hergestellt.

### Produktion der Enzyme

Für die rekombinante Enzymproduktion in einem *Escherichia coli*-Stamm wird zunächst das zu exprimierende Gen in einer PCR-Reaktion unter der Verwendung der genomischen DNA oder deren synthetisch an die Codon-Verwendung von *E. coli* angepasstes Äquivalent als Matrize zusammen mit spezifischen Oligonukleotiden, die zusätzlich Erkennungssequenzen für Restriktionsendonukleasen tragen, amplifiziert und aus dem Reaktionsgemisch isoliert. Nach dem Nukleinsäure-Verdau mit Restriktionsenzymen SphI und HindIII wird das für das Target-Enzym kodierende Genfragment in das mit SphI und HindIII geschnittene Rückgrat des Expressionsvektors pQE70-Kan ligiert. Das Ligationsprodukt wird in chemisch kompetente *E. coli*-Zellen Top10F transformiert und die entstandenen Kolonien werden für die Plasmid-Isolierung und Restriktionsanalyse benutzt.

Das Ergebnis des Klonierungs-Schritts wird mittels Restriktionsenzym-Verdau und DNA-Sequenzierung überprüft. Das resultierende Konstrukt trägt das Target-Gen unter dem IPTG-induzierbaren T5-Promotor.

Für die Überexpression des Enzymes in *E. coli* wird das resultierende Expressionsplasmid in die kompetenten Expressionszellen RB791 transformiert. Nach 24 h Inkubation bei 37 °C werden entstandene Kolonien für die Expressionstest in Luria-Bertani-Medium angeimpft.

Am nächsten Tag werden damit Expressionskulturen mit einer optische Dichte OD₅₅₀ von 0,02 angeimpft und bei 37 °C geschüttelt, bis eine OD₅₅₀ von 0,3 erreicht wurde. Anschließend wird die Temperatur auf 25 °C gesenkt und die Kulturen werden beim Erreichen einer OD₅₅₀ von 0,5 mit 0,1 mM IPTG induziert. Nach 22 h werden die Kulturen geerntet (vom Medium mittels Zentrifugation in Form eines Zellpellets abgetrennt) und auf die Expression des rekombinanten Enzymes mit Hilfe von SDS-Gelelektrophorese und einer Aktivitätsbestimmung (Verwendung in Use-Test oder optischenzymatischer Assay) analysiert. Die rekombinante Expression des Target-Enzymes erreicht meistens 50 % des *E. coli*-Gesamtproteins.

### Herstellung von Zell-Suspensionen

Zur Herstellung von Zell-Suspensionen wurden die nach obigen Verfahren hergestellten Zellpellets in einem geeigneten Gefäß eingewogen und mit Puffer versetzt (siehe Tabelle 1 für verwendete Puffer-Systeme) und unter Rührung im Eisbad gelöst. Der Massenanteil an Biomasse beträgt üblicherweise 20%, der Rest entfällt auf den Puffer.

### Herstellung von Homogenaten mittels Sonifier-Aufschluss

Der oben hergestellten Zell-Suspension wurde Lysozym in einer Menge von 0,5 mg/ml zugefügt. Zum Zell-Aufschluss wurde ein Branson Sonifier 450 verwendet. Die Suspension wurde in ein 5 ml Eppendorf-Vial überführt. Die Metallspitze des Apparats wurde nun in die Suspension getaucht, danach wurde die Suspension dreimal mit je 15 Ultraschall-Stößen (Einstellungen am Gerät: Timer = 15; Duty Cycle = 50; Output Control = 3 - 5) behandelt. Dazwischen wurde die Suspension 15 min im Eisbad inkubiert. Auf diese Weise wurde das Homogenat als Mischung von aufgeschlossenen Zellen und Puffer erhalten.

### Herstellung von Lysaten durch Zentrifugation

Das Homogenat wurde 10 min lang bei 4 °C und 16000 rpm zentrifugiert (Eppendorf Zentrifuge 5417R), um die unlöslichen Zellfragmente abzutrennen und das Lysat zu erhalten.

**Tabelle 1. Spenderorganismen und Aufschlussbedingungen für die verwendeten Enzyme.**

| **Enzymtyp (EC-Klasse)** | **katalysierte Reaktion(en)** | **Spenderorganismus** | **Aufschlussbedingungen** | **Literatur** |
|---|---|---|---|---|
| Dehydratase (EC 4.2.1.25) | Gluconat → KDG; Galactonat → KDGal | *Azotobacter vinelandii* DJ | Sonifier; 20% Biomasse in 100 mM TEA-HCI, pH 7 + 2,5 mM MgCl₂ | (Setubal et al., 2009); (NLM Protein database: AC081022.1) |
| Aldolase (EC 4.1.2.55) | KDG / KDGal → Pyruvat + D-Glyceraldehyd | *Sulfolobus acidocaldarius* | Sonifier; 20% Biomasse in 100 mM TEA-HCI, pH 7 | Wolterink-van Loo et al., 2009) |
| D-Xylose-Reduktase (EC 1.1.1.307) | D-Glyceraldehyd → Glycerin | *Debaryomyces hansenii* | Sonifier; 20% Biomasse in 100 mM TEA-HCI, pH 7 | (de Faria et al., 2009) |
| L-Lactat-Dehydrogenase (EC 1.1.1.27) | Pyruvat → L-Lactat | *Oryctolagus cuniculi* (Kaninchen muskel) | Sonifier; 50% Biomasse in 50 mM HEPES, pH 7,5 | (Zheng et al., 2004) |
| Alkoholdehydrog enase (NADP) (EC 1.1.1.2) | Isopropanol → Aceton | *Thermoanaerobium brockii* | Sonifier; 20% Biomasse in 100 mM TEA-HCI, pH 7 + 0,5 mM ZnCl₂ | (Lamed & Zeikus, 1980) |
| Alkoholdehydrog enase (NAD) (EC 1.1.1.1) | Isopropanol → Aceton | *Acetobacter pasteurianus* | Sonifier; 20% Biomasse in 100 mM TEA-HCI, pH 7 | (Chinnawirotpisa n et al., 2003) |

### Analytische Methoden

### High Performance Anion Exchange Chromatographv

Substrat-Umsätze und Produkt-Konzentrationen wurden durch HPAEC (High Performance Anion Exchange Chromatography) ermittelt. Dazu wurde ein Dionex ICS6000 System mit AS-AP Autosampler verwendet. Die Messung der organischen Säuren bzw. deren Anionen (Gluconat, KDG, KDGal, Pyruvat und Lactat) erfolgte mittels Leitfähigkeitsdetektion (CD) gekoppelt an einen Dionex AERS 500 elektrolytisch regenerierten Suppressor im externen Wassermodus. Zur Trennung der Analyten wurde eine Dionex IonPac AS11-HC-4µm Säule mit entsprechender Vorsäule sowie einem NaOH-Gradienten verwendet. Das Laufmittel wurde zusätzlich mit einer Dionex ATC Anion Trap Column vorbehandelt.

### High Performance Liquid Chromatography

Zur Quantifizierung von D-Glyceraldehyd und Glycerin mittels HPLC (High Performance Liquid Chromatography) wurde ein Agilent HPLC 1260 Infinity II Series System verwendet. Detektion erfolgt mittels eines Brechungsindex-Detektors (RI-Detektion). Zur Messung wird eine Phenomenex Rezex ROA-Organic Acid H+ (8%) Säule mit entsprechender Vorsäule verwendet und mit 1 mM Schwefelsäure isokratisch eluiert.

### Bestimmung von Enzym-Aktivitäten (optisch-enzvmatischer Assav)

Enzym-Aktivitäten in den Homogenaten bzw. Lysaten wurden mit einem Shimadzu UV-1900 Spektrophotometer bestimmt. Dazu wurde die Bildung bzw. der Verbrauch von NAD(P)H bei einer Wellenlänge von 340 nm über die Änderung der Absorption verfolgt. Die Messungen wurden mit 0,2 mM Kofaktor (NAD(P)⁺ bzw. NAD(P)H) durchgeführt. Dazu wurden 20 µl einer 10 mM Stock-Lösung des Kofaktors in einer Küvette (Greiner bio-one Semi-Micro-Küvette aus Polystyrol) vorgelegt und der gewünschte pH-Wert wurde mit 100 mM TEA-HCl-Puffer eingestellt (870 µl). Nach Temperieren der Küvette wurden 10 µl Homogenat bzw. Lysat (verdünnt oder unverdünnt) und 100 µl Substrat-Lösung zugesetzt und die Messung unmittelbar danach gestartet. Die Messungen wurden standardmäßig bei 25 °C durchgeführt. Das Homogenat bzw. Lysat wurde so verdünnt, dass die Änderung der Absorption bei 340 nm über eine Minute ca. 0,11 Absorptionseinheiten beträgt. Über den Extinktionskoeffizienten von NADH bzw. NADPH bei 340 nm (*ε* = 6220 L mol⁻¹ cm⁻¹) kann die Enzym-Aktivität des Lysats in U/ml (bezogen auf das Volumen des Lysats) bzw. U/g (bezogen auf die zur Herstellung eingesetzte Biomasse) bestimmt werden. 1 U steht dabei für 1 µmol Substratumsatz pro Minute (1 U = 1 µmol/min = 1,67·10⁻⁸ kat).

**Tabelle 2. Aktivitätsparameter für die Alkoholdehydrogenasen (NAD- bzw. NADP-spezifisch), die D-Xylose-Reduktase sowie die L-Lactat-Dehydrogenase (L-LacDH)**

| **Enzym** | **Aktivität Lysat** | **Substrat, Kofaktor** | **Puffer** |
|---|---|---|---|
| Alkoholdehydrogenase (NADP) | 240 U/ml | 6 v% Isopropanol, NADP⁺ | TEA-HCl pH 8,5 |
| Alkoholdehydrogenase (NAD) | 230 U/ml | 6 v% Isopropanol, NAD⁺ | |
| D-Xylose-Reduktase | 800 U/ml | 10 mM D-Glyceraldehyd, NADPH | |
| L-LacDH | 950 U/ml | 10 mM Natrium-Pyruvat, NADH | |

### Allgemeines zur Aufarbeitung der Reaktionslösung

Die erfindungsgemäß hergestellte Glycerin- und Lactat-haltige bzw. Glycerin- und Pyruvat-haltige wässrige Lösung wird erhalten, indem nach Beendigung der Reaktion die Zellbestandteile durch Denaturieren (z.B. durch Einwirkung von Wärme) und anschließender Filtration bzw. Zentrifugation entfernt werden. Zur Entfernung von kleineren Zellbestandteilen kann zusätzlich eine Membranfiltration durchgeführt werden. Das so erhaltene Filtrat kann nun beispielsweise durch Verdampfung des Wassers zur gewünschten Konzentration aufkonzentriert (z.B. auf 250 g/l Glycerin oder Lactat) werden.

Mit den nachfolgenden Beispielen werden bevorzugte Varianten des erfindungsgemäßen Verfahrens noch näher beschrieben. Die in diesen Beispielen eingesetzten Suspensionen, Homogenate und Lysate wurden nach den oben beschriebenen Verfahren hergestellt.

### Beispiel 1

### Umsatz von Natrium-Gluconat zu Pyruvat und Glycerin

In einem 2 ml Glas-Vial wurden folgende Komponenten vorgelegt: 25 µl eines 2000 mM TEA-HCl-Puffers (pH 8,5), 143 µl deionisiertes Wasser, 80 µl Dehydratase-Lysat, 80 µl Aldolase-Lysat, 10 µl D-Xylose-Reduktase-Lysat, welches zuvor 1:4 in 100 mM TEA-HCl-Puffer (pH 7) verdünnt worden war (entspricht einer Aktivität von 2 U), sowie 11 µl Alkoholdehydrogenase-Lysat (NADP-spezifisch; entspricht einer Aktivität von 2 U). Durch Zugabe von 100 µl einer Natrium-Gluconat-Lösung (2296 mM) wurde die Reaktion gestartet. Zur Kofaktor-Regeneration wurden 50 µl Isopropanol zugesetzt. Der Ansatz wurde insgesamt 48 h bei 30 °C unter kontinuierlichem Schütteln (1200 rpm, Eppendorf Thermomixer) inkubiert. 26 h nach Start der Reaktion wurden 100 µl einer Isopropanol/Wasser-Mischung (3/7 v/v) zum Ansatz zugesetzt. Das Gesamtvolumen des Ansatzes bei Start der Reaktion betrug 500 µl.

Zur Aufarbeitung wurden nach Ausgleich des verdunsteten Volumens 20 µl des Ansatzes mit 180 µl Reinstwasser und 200 µl MeOH versetzt und bei 60 °C für 20 min inkubiert (1200 rpm, Eppendorf Thermomixer). Nach der Denaturierung wurde die Probe 10 min bei max. g zentrifugiert. Der klare Überstand wurde 1:250 verdünnt und mittels HPAEC (Leitfähigkeitsdetektion) vermessen. Für die HPLC wurden 150 µl des klaren Überstands in HPLC-Vials überführt und vermessen (RI-Detektion). Die Ergebnisse sind im Folgenden tabellarisch dargestellt.

| | | Konzentration [mM] | Umsatz nach 48 h [%] |
|---|---|---|---|
| Substrat (t = 0 h) | Natrium-Gluconat | 459 | 64 |
| | | | |
| Produkte (t = 48 h) | Glycerin | 120 | 26 |
| | Pyruvat | 39 | 8 |

Zusätzlich zu den in der Tabelle angeführten Produkten werden auch die Intermediate KDG und KDGal detektiert.

Der obenstehenden Tabelle kann entnommen werden, dass Gluconat vom Enzymsystem zu Glycerin und Pyruvat umgesetzt wird.

### Beispiel 2

### Umsatz von Natrium-Gluconat zu Lactat und Glycerin

In einem 2 ml Glas-Vial (Vial 1) wurden folgende Komponenten vorgelegt: 25 µl eines 2000 mM TEA-HCl-Puffers (pH 8,5), 172 µl deionisiertes Wasser, 80 µl Dehydratase-Lysat, 80µl Aldolase-Lysat, 10 µl D-Xylose-Reduktase-Lysat, welches zuvor 1:4 in 100 mM TEA-HCl-Puffer (pH 7) verdünnt worden war (entspricht einer Aktivität von 2 U), 11 µl Alkoholdehydrogenase-Lysat (NADP-spezifisch; entspricht einer Aktivität von 2 U), 10 µl Alkoholdehydrogenase-Lysat (NAD-spezifisch; entspricht einer Aktivität von 2 U) sowie 11 µl Lactat-Dehydrogenase-Lysat, welches zuvor 1:5 in 100 mM TEA-HCl-Puffer (pH 7) verdünnt worden war (entspricht einer Aktivität von 2 U). In einem weiteren 2 ml Glas-Vial (Vial 2) wurde eine ähnliche Mischung, allerdings mit 122 µl (Vial 2) deionisiertem Wasser, vorgelegt.

Durch Zugabe von 50 µl einer Natrium-Gluconat-Lösung (2296 mM) zu Vial 1 (Vial 2: 100 µl) wurden die Reaktionen gestartet. Zur Kofaktor-Regeneration wurden je 50 µl Isopropanol zugesetzt. Die Ansätze wurden insgesamt 48 h bei 30 °C unter kontinuierlichem Schütteln (1200 rpm, Eppendorf Thermomixer) inkubiert. 26 h nach Start der Reaktion wurden je 100 µl einer Isopropanol/Wasser-Mischung (3/7 v/v) zu den Ansätzen zugesetzt. Das Gesamtvolumen der Ansätze bei Start der Reaktion betrug je 500 µl.

Zur Aufarbeitung wurden nach Ausgleich des verdunsteten Volumens x µl des Ansatzes mit *y* µl Reinstwasser (*x* = 40, *y* = 160 für Vial 1; *x* = 20, *y* = 180 für Vial 2) und 200 µl MeOH versetzt und bei 60 °C für 20 min inkubiert (1200 rpm, Eppendorf Thermomixer). Nach der Denaturierung wurden die Proben 10 min bei max. g zentrifugiert. Der klare Überstand wurde 1:250 verdünnt und mittels HPAEC (Leitfähigkeitsdetektion) vermessen. Für die HPLC wurden 150 µl des klaren Überstands in HPLC-Vials überführt und vermessen (RI-Detektion). Die Ergebnisse sind im Folgenden tabellarisch dargestellt.

| Vial 1 - 230 mM Substrat | | Konzentration [mM] | Umsatz nach 48 h [%] |
|---|---|---|---|
| Substrat (*t* = 0 h) | Natrium-Gluconat | 230 | 58 |
| | | | |
| Produkte (*t* = 48 h) | Glycerin | 131 | 57 |
| | Lactat | 137 | 60 |

| Vial 2 - 459 mM Substrat | | Konzentration [mM] | Umsatz nach 48 h [%] |
|---|---|---|---|
| Substrat (*t* = 0 h) | Natrium-Gluconat | 459 | 49 |
| | | | |
| Produkte (*t* = 48 h) | Glycerin | 191 | 42 |
| | Lactat | 190 | 41 |

Zusätzlich zu den in der Tabelle angeführten Produkten werden auch die Intermediate KDG und KDGal detektiert.

Den obenstehenden Tabellen kann entnommen werden, dass das Verfahren auch für den Umsatz einer konzentrierteren Substrat-Lösung (459 mM) geeignet ist.

### Beispiel 3

### Einfluss von verschiedenen Enzym-Formulierungen (Suspension, Homogenat bzw. Lysat) auf den Umsatz von Natrium-Gluconat zu Lactat und Glycerin

In einem 2 ml Glas-Vial wurden folgende Komponenten vorgelegt: 25 µl eines 2000 mM TEA-HCl-Puffers (pH 8,5), 130 µl deionisiertes Wasser, 80 µl Dehydratase-Formulierung (siehe nachfolgende Tabellen), 80 µl Aldolase-Formulierung, 10 µl D-Xylose-Reduktase-Formulierung, welche zuvor 1:4 in 100 mM TEA-HCl-Puffer (pH 7) verdünnt worden war (entspricht einer Aktivität von 2 U), 8 µl Alkoholdehydrogenase-Formulierung (NADP-spezifisch; entspricht einer Aktivität von 2 U), 9 µl Alkoholdehydrogenase-Formulierung (NAD-spezifisch; entspricht einer Aktivität von 2 U) sowie 8 µl Lactat-Dehydrogenase-Lysat, welches zuvor 1:5 in 100 mM TEA-HCl-Puffer (pH 7) verdünnt worden war (entspricht einer Aktivität von 2 U). Durch Zugabe von je 100 µl einer Natrium-Gluconat-Lösung (1145 mM) wurden die Reaktionen gestartet. Zur Kofaktor-Regeneration wurden je 50 µl Isopropanol zugesetzt. Die Ansätze wurden insgesamt 48 h bei 30 °C unter kontinuierlichem Schütteln (1200 rpm, Eppendorf Thermomixer) inkubiert. 26 h nach Start der Reaktion wurden je 100 µl einer Isopropanol/Wasser-Mischung (3/7 v/v) zu den Ansätzen zugesetzt. Das Gesamtvolumen der Ansätze bei Start der Reaktion betrug je 500 µl.

Zur Aufarbeitung wurden nach Ausgleich des verdunsteten Volumens 40 µl des Ansatzes mit 160 µl Reinstwasser und 200 µl MeOH versetzt und bei 60 °C für 20 min inkubiert (1200 rpm, Eppendorf Thermomixer). Nach der Denaturierung wurden die Proben 10 min bei max. g zentrifugiert. Der klare Überstand wurde 1:250 verdünnt und mittels HPAEC (Leitfähigkeitsdetektion) vermessen. Für die HPLC wurden 150 µl des klaren Überstands in HPLC-Vials überführt und vermessen (RI-Detektion). Die Ergebnisse sind im Folgenden tabellarisch dargestellt.

| Aldolase als Suspension; L-LacDH als Lysat; restliche Enzyme als Homogenat | | Konzentration [mM] | Umsatz nach 48 h [%] |
|---|---|---|---|
| Substrat (*t* = 0 h) | Natrium-Gluconat | 229 | 54 |
| | | | |
| Produkte (*t* = 48 h) | Glycerin | 92 | 40 |
| | Lactat | 80 | 35 |

| Aldolase als Suspension; restliche Enzyme als Lysat | | Konzentration [mM] | Umsatz nach 48 h [%] |
|---|---|---|---|
| Substrat (*t* = 0 h) | Natrium-Gluconat | 229 | 58 |
| | | | |
| Produkte (*t* = 48 h) | Glycerin | 90 | 39 |
| | Lactat | 76 | 33 |

| L-LacDH als Lysat; restliche Enzyme als Homogenat | | Konzentration [mM] | Umsatz nach 48 h [%] |
|---|---|---|---|
| Substrat (*t* = 0 h) | Natrium-Gluconat | 229 | 55 |
| | | | |
| Produkte (*t* = 48 h) | Glycerin | 102 | 45 |
| | Lactat | 80 | 35 |

| alle Enzyme als Lysat | | Konzentration [mM] | Umsatz nach 48 h [%] |
|---|---|---|---|
| Substrat (*t* = 0 h) | Natrium-Gluconat | 229 | 55 |
| | | | |
| Produkte (*t* = 48 h) | Glycerin | 107 | 47 |
| | Lactat | 98 | 43 |

Zusätzlich zu den in der Tabelle angeführten Produkten werden auch die Intermediate KDG und KDGal sowie Pyruvat detektiert.

Den obenstehenden Tabellen kann entnommen werden, dass unterschiedliche Enzym-Formulierungen (Suspensionen, Homogenate und Lysate) für das Enzym-System verwendet werden können. Den größten Umsatz erhält man, wenn alle Enzyme in Form von Lysaten zugesetzt werden.

### Beispiel 4

### Einfluss von Kofaktor-Zusatz (NAD⁺ und NADP⁺) auf den Umsatz von Natrium-Gluconat zu Lactat und Glycerin

In einem 2 ml Glas-Vial (Vial 1) wurden folgende Komponenten vorgelegt: 25 µl eines 2000 mM TEA-HCl-Puffers (pH 8,5), 130 µl deionisiertes Wasser, 80 µl Dehydratase-Lysat, 80 µl Aldolase-Lysat, 10 µl D-Xylose-Reduktase-Lysat, welches zuvor 1:4 in 100 mM TEA-HCl-Puffer (pH 7) verdünnt worden war (entspricht einer Aktivität von 2 U), 8 µl Alkoholdehydrogenase-Lysat (NADP-spezifisch; entspricht einer Aktivität von 2 U), 9 µl Alkoholdehydrogenase-Lysat (NAD-spezifisch; entspricht einer Aktivität von 2 U) sowie 8 µl Lactat-Dehydrogenase-Lysat, welches zuvor 1:5 in 100 mM TEA-HCI-Puffer (pH 7) verdünnt worden war (entspricht einer Aktivität von 2 U). In einem zweiten 2 ml Glas-Vial wurde eine ähnliche Mischung, allerdings mit 10 µl einer 10 mM NAD⁺-Lösung, 10 µl einer 10 mM NADP⁺-Lösung sowie 110 µl statt 130 µl deionisiertes Wasser, vorgelegt.

Durch Zugabe von je 100 µl einer Natrium-Gluconat-Lösung (1145 mM) zu beiden Glas-Vials wurden die Reaktionen gestartet. Zur Kofaktor-Regeneration wurden je 50 µl Isopropanol zugesetzt. Die Ansätze wurden insgesamt 48 h bei 30 °C unter kontinuierlichem Schütteln (1200 rpm, Eppendorf Thermomixer) inkubiert. 26 h nach Start der Reaktion wurden je 100 µl einer Isopropanol/Wasser-Mischung (3/7 v/v) zu den Ansätzen zugesetzt. Das Gesamtvolumen der Ansätze bei Start der Reaktion betrug je 500 µl.

Zur Aufarbeitung wurden nach Ausgleich des verdunsteten Volumens 40 µl des Ansatzes mit 160 µl Reinstwasser und 200 µl MeOH versetzt und bei 60 °C für 20 min inkubiert (1200 rpm, Eppendorf Thermomixer). Nach der Denaturierung wurden die Proben 10 min bei max. g zentrifugiert. Der klare Überstand wurde 1:250 verdünnt und mittels HPAEC (Leitfähigkeitsdetektion) vermessen. Für die HPLC wurden 150 µl des klaren Überstands in HPLC-Vials überführt und vermessen (RI-Detektion). Die Ergebnisse sind im Folgenden tabellarisch dargestellt.

| Vial 1 - kein Kofaktor-Zusatz | | Konzentration [mM] | Umsatz nach 48 h [%] |
|---|---|---|---|
| Substrat (*t* = 0 h) | Natrium-Gluconat | 229 | 55 |
| | | | |
| Produkte (*t* = 48 h) | Glycerin | 107 | 47 |
| | Lactat | 98 | 43 |

| Vial 2 - Kofaktor-Zusatz (0,2 mM NAD⁺ und 0,2 mM NADP⁺ final) | | Konzentration [mM] | Umsatz nach 48 h [%] |
|---|---|---|---|
| Substrat (*t* = 0 h) | Natrium-Gluconat | 229 | 53 |
| | | | |
| Produkte (*t* = 48 h) | Glycerin | 103 | 45 |
| | Lactat | 100 | 43 |

Zusätzlich zu den in der Tabelle angeführten Produkten werden auch die Intermediate KDG und KDGal sowie Pyruvat detektiert.

Den obenstehenden Tabellen kann entnommen werden, dass die Zugabe von Kofaktor (0,2 mM NAD⁺ und 0,2 mM NADP⁺) bei einer Natrium-Gluconat-Konzentration von 229 mM keine Vorteile bezüglich des Umsatzes mit sich bringt, da bereits ausreichend Kofaktor aus den eingesetzten Lysaten vorliegt. Bei höheren Substrat-Konzentrationen kann allerdings ein Kofaktor-Zusatz notwendig sein.

### Beispiel 5

### Umsatz von Kalium-Galactonat zu Pyruvat und Glycerin

In einem 2 ml Glas-Vial wurden folgende Komponenten vorgelegt: 25 µl eines 2000 mM TEA-HCl-Puffers (pH 8,5), 147 µl deionisiertes Wasser, 80 µl Dehydratase-Lysat, 80 µl Aldolase-Lysat, 10 µl D-Xylose-Reduktase-Lysat, welches zuvor 1:4 in 100 mM TEA-HCl-Puffer (pH 7) verdünnt worden war (entspricht einer Aktivität von 2 U), sowie 8 µl Alkoholdehydrogenase-Lysat (NADP-spezifisch; entspricht einer Aktivität von 2 U). Durch Zugabe von 100 µl einer Kalium-Galactonat-Lösung (1282 mM) wurde die Reaktion gestartet. Zur Kofaktor-Regeneration wurden 50 µl Isopropanol zugesetzt. Der Ansatz wurde insgesamt 48 h bei 30 °C unter kontinuierlichem Schütteln (1200 rpm, Eppendorf Thermomixer) inkubiert. 26 h nach Start der Reaktion wurden 100 µl einer Isopropanol/Wasser-Mischung (3/7 v/v) zur Probe zugesetzt. Das Gesamtvolumen des Ansatzes bei Start der Reaktion betrug 500 µl.

Zur Aufarbeitung wurden nach Ausgleich des verdunsteten Volumens 40 µl des Ansatzes mit 160 µl Reinstwasser und 200 µl MeOH versetzt und bei 60 °C für 20 min inkubiert (1200 rpm, Eppendorf Thermomixer). Nach der Denaturierung wurde die Probe 10 min bei max. g zentrifugiert. Der klare Überstand wurde 1:250 verdünnt und mittels HPAEC (Leitfähigkeitsdetektion) vermessen. Für die HPLC wurden 150 µl des klaren Überstands in HPLC-Vials überführt und vermessen (RI-Detektion). Die Ergebnisse sind im Folgenden tabellarisch dargestellt.

| | | Konzentration [mM] | Umsatz nach 48 h [%] |
|---|---|---|---|
| Substrat (*t* = 0 h) | Kalium-Galactonat | 256 | 72 |
| | | | |
| Produkte (*t* = 48 h) | Glycerin | 53 | 21 |
| | Pyruvat | 24 | 10 |

Zusätzlich zu den in der Tabelle angeführten Produkten werden auch die Intermediate KDG und KDGal detektiert.

Der obenstehenden Tabelle kann entnommen werden, dass das Enzymsystem auch Galactonat zu Glycerin und Pyruvat umsetzt.

### Beispiel 6

### Umsatz von Kalium-Galactonat zu Lactat und Glycerin

In einem 2 ml Glas-Vial wurden folgende Komponenten vorgelegt: 25 µl eines 2000 mM TEA-HCl-Puffers (pH 8,5), 130 µl deionisiertes Wasser, 80 µl Dehydratase-Lysat, 80 µl Aldolase-Lysat, 10 µl D-Xylose-Reduktase-Lysat, welches zuvor 1:4 in 100 mM TEA-HCl-Puffer (pH 7) verdünnt worden war (entspricht einer Aktivität von 2 U), 8 µl Alkoholdehydrogenase-Lysat (NADP-spezifisch; entspricht einer Aktivität von 2 U), 9 µl Alkoholdehydrogenase-Lysat (NAD-spezifisch; entspricht einer Aktivität von 2 U) sowie 8 µl Lactat-Dehydrogenase-Lysat, welches zuvor 1:5 in 100 mM TEA-HCl-Puffer (pH 7) verdünnt worden war (entspricht einer Aktivität von 2 U). Durch Zugabe von 100 µl einer Kalium-Galactonat-Lösung (1282 mM) wurde die Reaktion gestartet. Zur Kofaktor-Regeneration wurden 50 µl Isopropanol zugesetzt. Der Ansatz wurde insgesamt 48 h bei 30 °C unter kontinuierlichem Schütteln (1200 rpm, Eppendorf Thermomixer) inkubiert. 26 h nach Start der Reaktion wurden 100 µl einer Isopropanol/Wasser-Mischung (3/7 v/v) zur Probe zugesetzt. Das Gesamtvolumen des Ansatzes bei Start der Reaktion betrug 500 µl.

Zur Aufarbeitung wurden nach Ausgleich des verdunsteten Volumens 40 µl des Ansatzes mit 160 µl Reinstwasser und 200 µl MeOH versetzt und bei 60 °C für 20 min inkubiert (1200 rpm, Eppendorf Thermomixer). Nach der Denaturierung wurde die Probe 10 min bei max. g zentrifugiert. Der klare Überstand wurde 1:250 verdünnt und mittels HPAEC (Leitfähigkeitsdetektion) vermessen. Für die HPLC wurden 150 µl des klaren Überstands in HPLC-Vials überführt und vermessen (RI-Detektion). Die Ergebnisse sind im Folgenden tabellarisch dargestellt.

| | | Konzentration [mM] | Umsatz nach 48 h [%] |
|---|---|---|---|
| Substrat (*t* = 0 h) | Kalium-Galactonat | 256 | 70 |
| | | | |
| Produkte (*t* = 48 h) | Glycerin | 84 | 33 |
| | Lactat | 57 | 22 |

Zusätzlich zu den in der Tabelle angeführten Produkten werden auch die Intermediate KDG und KDGal sowie Pyruvat detektiert.

Der obenstehenden Tabelle kann entnommen werden, dass das Enzymsystem auch Galactonat zu Glycerin und Lactat umsetzt.

### Literatur

Wee, Y.-J., Kim, J.-N., & Ryu, H.-W. (2006). Biotechnological Production of Lactic Acid and Its Recent Applications. Food Technology & Biotechnology, 44(2), 163-172. https://www.ftb.com.hr/80-volume-44-issue-no-2/445-biotechnological-production-of-lactic-acid-and-its-recent-applications Smith, W. P. (1996). Epidermal and dermal effects of topical lactic acid. Journal of the American Academy of Dermatology, 35(3), 388-391. https://doi.org/10.1016/s0190-9622(96)90602-7
Balla, E., Daniilidis, V., Karlioti, G., Kalamas, T., Stefanidou, M., Bikiaris, N. D., Vlachopoulos, A., Koumentakou, I., Bikiaris, D. N. (2021). Poly(lactic Acid): A Versatile Biobased Polymer for the Future with Multifunctional Properties-From Monomer Synthesis, Polymerization Techniques and Molecular Weight Increase to PLA Applications. Polymers, 13(11), 1822. https://doi.org/10.3390/polym13111822
Sutiono, S., Teshima, M., Beer, B., Schenk, G., & Sieber, V. (2020). Enabling the Direct Enzymatic Dehydration of d-Glycerate to Pyruvate as the Key Step in Synthetic Enzyme Cascades Used in the Cell-Free Production of Fine Chemicals. ACS Catalysis, 10(5), 3110-3118. https://doi.org/10.1021/acscatal.9b05068
Prestes, P. S., de Oliveira, M. M. M., & Leonardi, G. R. (2013). Randomized clinical efficacy of superficial peeling with 85% lactic acid versus 70% glycolic acid. Anais brasileiros de dermatologia, 88(6), 900-905. https://doi.org/10.1590/abd1806-4841.20131888
O'Connor, A. A., Lowe, P. M., Shumack, S., & Lim, A. C. (2018). Chemical peels: A review of current practice. Australasian Journal of Dermatology, 59(3), 171-181. https://doi.org/10.1111/ajd.12715
Ghersetich, I., Brazzini, B., Peris, K., Cotellessa, C., Manunta, T. & Lotti, T. (2004). Pyruvic Acid Peels for the Treatment of Photoaging. Dermatologic Surgery, 30(1), 32-36. https://doi.org/10.1111/j.1524-4725.2004.30002.x
*Apotheke des Universitätsklinikums Heidelberg* (17.07.2015). Pflegesalbe 100g. Verfügbar unter: https://www.klinikum.uni-heidelberg.de/fileadmin/apotheke/Rezeptur-Vorschriften/Pflegesalbe.pdf (Zugriff am 24.10.2022)
Tan, H. W., Abdul Aziz, A. R., & Aroua, M. K. (2013). Glycerol production and its applications as a raw material: A review. Renewable and Sustainable Energy Reviews, 27, 118-127. https://doi.org/10.1016/j.rser.2013.06.035
Rodrigues, A., Bordado, J. C., & dos Santos, R. G. (2017). Upgrading the Glycerol from Biodiesel Production as a Source of Energy Carriers and Chemicals-A Technological Review for Three Chemical Pathways. Energies, 10(11), 1817. https://doi.org/10.3390/en10111817
Wang, Z. X., Zhuge, J., Fang, H., & Prior, B. A. (2001). Glycerol production by microbial fermentation: a review. Biotechnology Advances, 19(3), 201-223. https://doi.org/10.1016/s0734-9750(01)00060-x
Aslankoohi, E., Rezaei, M. N., Vervoort, Y., Courtin, C. M., & Verstrepen, K. J. (2015). Glycerol Production by Fermenting Yeast Cells Is Essential for Optimal Bread Dough Fermentation. PLOS ONE, 10(3), e0119364. https://doi.org/10.1371/journal.pone.0119364
IRENA & METHANOL INSTITUTE (2021). Innovation Outlook: Renewable Methanol, International Renewable Energy Agency, Abu Dhabi. https://www.irena.org/publications/2021/Jan/Innovation-Outlook-Renewable-Methanol (Zugriff am 19.10.2022)
Hromádková, Z., & Ebringerová, A. (1995). Isolation and Characterization of Hemicelluloses of Corn Hulls. Chemical Papers 49(2), 97-101. https://chempap.org/?id=7&paper=3556
Collins, S. R. A., Wellner, N., Martinez Bordonado, I., Harper, A. L., Miller, C. N., Bancroft, I., & Waldron, K.W. (2014). Variation in the chemical composition of wheat straw: the role of tissue ratio and composition. Biotechnology for Biofuels, 7, 121. https://doi.org/10.1186/s13068-014-0121-y
Kornecki, J. F., Carballares, D., Tardioli, P. W., Rodrigues, R. C., Berenguer-Murcia, A., Alcántara, A. R., & Fernandez-Lafuente, R. (2020). Enzyme production of D-gluconic acid and glucose oxidase: successful tales of cascade reactions. Catalysis Science & Technology, 10(17), 5740-5771. https://doi.org/10.1039/D0CY00819B
Entner, N., & Doudoroff, M. (1952). GLUCOSE AND GLUCONIC ACID OXIDATION OF PSEUDOMONAS SACCHAROPHILA. Journal of Biological Chemistry, 196(2), 853-862. https://doi.org/10.1016/S0021-9258(19)52415-2
Conway T. (1992). The Entner-Doudoroff pathway: history, physiology and molecular biology. FEMS Microbiology Reviews, 9(1), 1-27. https://doi.org/10.1111/j.1574-6968.1992.tb05822.x
Peekhaus, N., & Conway, T. (1998). What's for Dinner?: Entner-Doudoroff Metabolism in Escherichia coli. Journal of Bacteriology, 180(14), 3495-3502. https://doi.org/10.1128/JB.180.14.3495-3502.1998
Reher, M., Fuhrer, T., Bott, M., & Schönheit, P. (2010). The Nonphosphorylative Entner-Doudoroff Pathway in the Thermoacidophilic Euryarchaeon Picrophilus torridus Involves a Novel 2-Keto-3-Deoxygluconate-Specific Aldolase. Journal of Bacteriology, 192(4), 964-974. https://doi.org/10.1128/JB.01281-09
Sutter, J. M., Tästensen, J. B., Johnsen, U., Soppa, J., & Schönheit, P. (2016). Key Enzymes of the Semiphosphorylative Entner-Doudoroff Pathway in the Haloarchaeon Haloferax volcanii: Characterization of Glucose Dehydrogenase, Gluconate Dehydratase, and 2-Keto-3-Deoxy-6-Phosphogluconate Aldolase. Journal of Bacteriology, 198(16), 2251-2262. https://doi.org/10.1128/JB.00286-16
Lamble, H. J., Heyer, N. I., Bull, S. D., Hough, D. W., & Danson, M. J. (2003). Metabolic Pathway Promiscuity in the Archaeon Sulfolobus solfataricus Revealed by Studies on Glucose Dehydrogenase and 2-Keto-3-deoxygluconate Aldolase. Journal of Biological Chemistry, 278(36), 34066-34072. https://doi.org/10.1074/jbc.M305818200
Theodossis, A., Walden, H., Westwick, E. J., Connaris, H., Lamble, H. J., Hough, D. W., Danson, M. J., & Taylor, G. L. (2004). The Structural Basis for Substrate Promiscuity in 2-Keto-3-deoxygluconate Aldolase from the Entner-Doudoroff Pathway in Sulfolobus solfataricus. Journal of Biological Chemistry, 279(42), 43886-43892. https://doi.org/10.1074/jbc.M407702200
Goldberg, K., Schroer, K., Lütz, S., & Liese, A. (2007). Biocatalytic ketone reduction-a powerful tool for the production of chiral alcohols-part I: processes with isolated enzymes. Applied Microbiology and Biotechnology, 76, 237-248. https://doi.org/10.1007/s00253-007-1002-0
Schrittwieser, J. H., Velikogne, S., Hall, M. & Kroutil, W. (2018). Artificial Biocatalytic Linear Cascades for Preparation of Organic Molecules. Chemical Reviews, 118(1), 270-348. https://doi.org/10.1021/acs.chemrev.7b00033
Lin, B., & Tao, Y. (2017). Whole-cell biocatalysts by design. Microbial Cell Factories, 16, 106. https://doi.org/10.1186/s12934-017-0724-7
Moore, S. & Link, K.P. (1940). CARBOHYDRATE CHARACTERIZATION: I. THE OXIDATION OF ALDOSES BY HYPOIODITE IN METHANOL II. THE IDENTIFICATION OF SEVEN ALDO-MONOSACCHARIDES AS BENZIMIDAZOLE DERIVATIVES. Journal of Biological Chemistry, 133, 293-311. https://doi.org/10.1016/S0021-9258(18)73312-7
Setubal, J. C., dos Santos, P., Goldman, B. S., Ertesvåg, H., Espin, G., Rubio, L. M., Valla, S., Almeida, N. F., Balasubramanian, D., Cromes, L., Curatti, L., Du, Z., Godsy, E., Goodner, B., Hellner-Burris, K., Hernandez, J. A., Houmiel, K., Imperial, J., Kennedy, C., Larson, T. J., Latreille, P., Ligon, L. S., Lu, J., Mærk, M., Miller, N. M., Norton, S., O'Carroll, I. P., Paulsen, I., Raulfs, E. C., Roemer, R., Rosser, J., Segura, D., Slater, S., Stricklin, S. L., Studholme, D. J., Sun, J., Viana, C. J., Wallin, E., Wang, B., Wheeler, C., Zhu, H., Dean, D. R., Dixon, R., & Wood, D. (2009). Genome sequence of Azotobacter vinelandii, an obligate aerobe specialized to support diverse anaerobic metabolic processes. Journal of Bacteriology, 191(14), 4534-4545. https://doi.org/10.1128/JB.00504-09
Protein [Internet]. Bethesda (MD): National Library of Medicine (US), National Center for Biotechnology Information. Accession No. ACO81022.1, L-arabonate dehydratase [Azotobacter vinelandii DJ]. Verfügbar unter: https://www.ncbi.nlm.nih.gov/protein/226721851 (Zugriff am 04.10.2022)
Wolterink-van Loo, S., Siemerink, M. A. J., Perrakis, G., Kaper, T., Kengen, S. W. M., & van der Oost, J. (2009). Improving low-temperature activity of Sulfolobus acidocaldarius 2-keto-3-deoxygluconate aldolase. Archaea, 2(4), 233-239. https://doi.org/10.1155/2009/194186
de Faria, J. T., Sampaio, F. C., Converti, A., Lopes Passos, F. M., Minim, V. P., & Minim, L. A. (2009). Use of response surface methodology to evaluate the extraction of Debaryomyces hansenii xylose reductase by aqueous two-phase system. Journal of Chromatography B: Analytical Technologies in the Biomedical and Life Sciences, 877(27), 3031-3037. https://doi.org/10.1016/j.jchromb.2009.07.023
Zheng, Y., Guo, S., Guo, Z., & Wang, X. (2004). Effects of N-terminal deletion mutation on rabbit muscle lactate dehydrogenase. Biochemistry (Moscow), 69(4), 401-406. https://doi.org/10.1023/b:biry.0000026195.31821.el
Lamed, R., & Zeikus, J. G. (1980). Ethanol production by thermophilic bacteria: relationship between fermentation product yields of and catabolic enzyme activities in Clostridium thermocellum and Thermoanaerobium brockii. Journal of Bacteriology, 144(2), 569-578. https://doi.org/10.1128/jb.144.2.569-578.1980
Chinnawirotpisan, P., Matsushita, K., Toyama, H., Adachi, O., Limtong, S., & Theeragool, G. (2003). Purification and characterization of two NAD-dependent alcohol dehydrogenases (ADHs) induced in the quinoprotein ADH-deficient mutant of Acetobacter pasteurianus SKU1108. Bioscience, Biotechnology, and Biochemistry, 67(5), 958-965. https://doi.org/10.1271/bbb.67.958

## Patentansprüche

1. Verfahren zur Herstellung einer wässerigen Lösung, welche Glycerin und ein Salz der Brenztraubensäure oder der Milchsäure enthält, indem ein Salz der Gluconsäure und/oder der Galactonsäure, in einer wässerigen, Alkohol-hältigen Lösung in vitro mit einem Enzymsystem, welches eine Dehydratase, eine Aldolase, eine Alkoholdehydrogenase samt Kofaktor, eine Glyceraldehyd-Reduktase samt Kofaktor und gegebenenfalls eine Lactat-Dehydrogenase samt Kofaktor umfasst, behandelt wird, wodurch eine Glycerin- und ein Salz der Brenztraubensäure oder der Milchsäure hältige wässerige Lösung erhalten wird, aus welcher das Enzymsystem abgetrennt und welche gegebenenfalls aufkonzentriert wird.

2. Verfahren nach Anspruch 1, **dadurch gekennzeichnet, dass** das Salz der Gluconsäure und/oder der Galactonsäure ein Alkalisalz ist und in der Alkohol-hältigen Lösung in einer Konzentration zwischen 50 und 350 g/l vorliegt.

3. Verfahren nach Anspruch 2, **dadurch gekennzeichnet, dass** das Alkalisalz in einer Konzentration zwischen 150 und 300 g/l vorliegt.

4. Verfahren nach Anspruch 3, **dadurch gekennzeichnet, dass** das Alkalisalz in einer Konzentration zwischen 150 und 250 g/l ist.

5. Verfahren nach einem der Ansprüche 1 bis 4, **dadurch gekennzeichnet, dass** der Alkohol in der Alkohol-hältigen Lösung Isopropanol ist.

6. Verfahren nach einem der Ansprüche 1 bis 5, **dadurch gekennzeichnet, dass** die das Enzymsystem bildenden Enzyme als Lysat der entsprechenden, sie bildenden Zellen vorliegen.

7. Verfahren nach einem der Ansprüche 1 bis 5, **dadurch gekennzeichnet, dass** die das Enzymsystem bildenden Enzyme als Homogenat der entsprechenden, sie bildenden Zellen vorliegen.

8. Verfahren nach einem der Ansprüche 1 bis 5, **dadurch gekennzeichnet, dass** die das Enzymsystem bildenden Enzyme als Suspension der entsprechenden, sie bildenden Zellen vorliegen.
